# EUROPEAN PATENT APPLICATION

(11) **EP 2 279 724 A2**
(43) Date of publication of application: **02.02.2011**
(21) Application number: 10168665.7
(22) Date of filing: 07.07.2010
(51) Int. Cl.: A61K 8/06, A61K 8/86, A61Q 19/00, B01F 17/42, B01F 17/44

(54) **Oil-in-water microemulsion carrying large amount of oil**

(30) Priority: 31.07.2009 CN 200910161461
(71) Applicant: Evonik Goldschmidt GmbH, 45127 Essen (DE)
(72) Inventor: Zhang, Haizhou, 201108, Shanghai (CN); Zou, Jiali, 201108, Shanghai (CN); Dai, Jingya, 201108, Shanghai (CN); Gao, Zhiheng, 201108, Shanghai (CN)

(57) **Abstract**

The present invention relates to an oil-in-water microemulsion, which comprises, on the basis of its total weight, the following components of A) 55-95 wt% of a water phase; B) 0.1-20 wt% of an oil phase, wherein the oil phase comprises at least one oil/fat component containing polyoxypropylene chains; and C) 1-25 wt% of an oil-in-water nonionic emulsifier, wherein the emulsifier is selected from the following group of: hydrophilic nonionic surfactants with polyoxyethylene chains as hydrophilic groups, polyglycerol fatty acid esters, polyglycerol fatty alcohol ethers, sucrose fatty acid esters, and hydrocarbyl polyglycosides. The oil-in-water microemulsion of the present invention carries a large amount of oil; when it is used as a rinse-off product, it would remove the colour makeup very rapidly, and it has a easy-to-wash cleansing effect; and when it is used as a leave-on product, it would provide a moisturizing feel and good skin feel different from ordinary aqueous products due to a relatively small amount of the emulsifier used. In addition, the present invention also relates to the use of an oil/fat containing polyoxypropylene chains in increasing the amount of oil carried in an oil-in-water microemulsion.

## Description

### Technical Field

The present invention relates to an oil-in-water microemulsion carrying a large amount of oil. More particularly, the present invention relates to a microemulsion, in which the oil phase contains oil/fat containing polyoxypropylene chains. In addition, the present invention also relates to the use of said oil/fat containing polyoxypropylene chains in increasing the amount of oil carried in an oil-in-water microemulsion.

### Background Art

When two or more than two immiscible liquids are mixed and emulsified, and then the average diameter of the dispersed liquid droplets or emulsified particles is between 5-100 nm, this system is referred to as a microemulsion. The microemulsion is a system which is normally formed by at least three components: water, oil and a surfactant. Macroscopically this system shows a homogenous phase. Since the dispersed micro liquid droplets have a poor light scattering capability, the microemulsion generally is in a translucent to transparent state. When the dispersion phase is an oil phase, and the dispersion medium is water, this microemulsion is referred to as an oil-in-water microemulsion; the other way aropund the microemulsion is referred to as a water-in-oil microemulsion. Thermodynamically, a microemulsion is a stable system which can be formed spontaneously without using special production equipment and processes. Microemulsions have unique appearances and special properties, and their applications in the industry for household chemicals are gaining more and more attention.

The particle size of the liquid droplets in a conventional emulsion is usually more than 1 µm, and the emulsion is opaque and milky white due to light scattering effects. The emulsion is unstable thermodynamically, is produced by external work, and thus the separation of oil and water phases occurs after being placed undisturbed for a period of time. However, in industrial applications, an emulsion can achieve the stability, i.e., a dynamic stability, for a long period of time by way of electrostatic repulsion, etc. Compared with the emulsion, the microemulsion when used for preparing cosmetics has the advantages as follows: 1) it is optically transparent, and any non-uniform or precipitated substance can be discovered easily; 2) the microemulsion can form spontaneously, and therefore it has the features of energy-saving and high efficiency; 3) it has a good stability, so it can be stored for a long period of time without delamination; 4) it has a good solubilization effect, and it can be prepared into a product having a high oil component without a greasy feel; and by way of the solubilization effect of the microemulsion, it can also improve the stability and effect of the active components and drugs; 5) the micelle particles are fine, and permeate into the skin easily; and 6) the microemulsion can also encapsulate nano particles such as TiO₂, ZnO, etc., and when added into the cosmetics, these particles have the features of whitening, absorbing ultra-violet rays and radiation of infrared ray, etc. Therefore, the microemulsion cosmetics are developing very rapidly in recent years, are well used in many fields for cosmetics, and have a very broad market prospect.

The microemulsion normally can only be formed by adding a relatively large amount of surfactant or emulsifier, and when the amount of the surfactant or the emulsifier is too small, it cannot be formed. In a traditional oil-in-water microemulsion, the content of the oil phase is generally quite low; in other words, the amount of the emulsifier used is relatively high. In the traditional oil-in-water microemulsion, the weight ratio of the oil phase to the oil-in-water nonionic emulsifier is between 0.1-0.25, and is generally lower than 0.2, such microemulsion products have an unpleasant sticky feel when they are used and will lead to more residual emulsifier. There is always a need for using as little emulsifier as possible to prepare the oil-in-water microemulsion products having an oil phase content as high as possible. In this way, not only skin stimulation by the surfactant or the emulsifier is reduced, but also the costs can be reduced. If a large amount of nonionic surfactant is used in the formulation, particularly a polyoxyethylene nonionic surfactant with repeating units of more than 10, it would also cause irritation to eyes.

### Contents of the invention

In view of the above-mentioned status of the prior art, the present inventor has carried out extensive and thorough researches on the oil-in-water microemulsion carrying a large amount of oil, and as a result has found that when an oil/fat containing polyoxypropylene chains is included in the oil phase, it cannot only increase the amount of oil carried, but also reduces the amount of the emulsifier used. When an oil-in-water microemulsion so obtained is used as a rinse-off product, it carries a large amount of oil and can remove colour makeup very rapidly, and it has an easy-to-wash cleansing effect; and when it is used as a leave-on product, the amount of the emulsifier used is small, and it provides a moisturizing feel and a good skin feel different from the ordinary aqueous products.

Therefore, an objective of the present invention is to provide an oil-in-water microemulsion carrying a large amount of oil.

The above objective is achieved by an oil-in-water microemulsion carrying a large amount of oil described below, and the oil-in-water microemulsion, on the basis of its total weight comprises the following components of:
A) 55-95 wt% of a water phase;
B) 0.1-20 wt% of an oil phase, wherein the oil phase comprises at least one oil/fat component containing polyoxypropylene chains; and
C) 1-25 wt% of an oil-in-water nonionic emulsifier, wherein the emulsifier is one or more hydrophilic nonionic surfactants selected from the following group of hydrophilic nonionic surfactants with polyoxyethylene chains as hydrophilic groups, polyglycerol fatty acid esters, polyglycerol fatty alcohol ethers, sucrose fatty acid esters, and hydrocarbyl polyglycosides.

In the present invention, the definition of the "amount of oil carried" is as follows: in the cases that the type and amount of the water phase and the emulsifier components used in the oil-in-water microemulsion system, and the type of the oil phase have been determined and the state of the microemulsion is maintained, the largest amount of the oil phase that can be emulsified in this microemulsion system. If this largest amount of the oil phase is exceeded, the system will become cloudy, and a microemulsion will not be formed. Said largest amount of the oil phase is referred to as the emulsion system's "amount of oil carried"; however, for each particular microemulsion system, the actual oil phase content is referred to as the "actual oil content" of the system.

### Component A

In the microemulsion of the present invention, it comprises a water phase which accounts for the most part, and this water phase is as a continuous phase.

The water phase of the microemulsion in the present invention mainly comprises water, and water generally accounts for at least 50 wt%, at least 60 wt%, at least 70 wt%, at least 80 wt%, at least 90 wt% or at least 95 wt% of the water phase.

According to the present invention, in addition to water the water phase can be added with an alcohol substance of a low molecular weight, like for example ethanol, glycerol, propanediol, 1,3-butanediol, etc. The amount of the alcohol substance of a low molecular weight used is generally 0-30 wt%, based on the total weight of the microemulsion of the present invention.

According to the present invention, other functional addictives can also be added in the water phase, such as hyaluronic acid, lactic acid, etc.

In addition, in the water phase of the present invention, polyethylene glycol fatty acid ester materials, such as polyethylene glycol-120 methylglucose dioleate can also be added for thickening effects. The amount of the thickener added is generally 0-10 wt%, based on the total weight of the microemulsion in the present invention.

In the microemulsion of the present invention, the amount of water phase used as the component A is generally 55-95 wt%, preferably 75-95 wt%, and more preferably 85-95 wt%, based on the total weight of the microemulsion.

### Component B

The microemulsion of the present invention comprises an oil phase, which is in a dispersion phase, and exists in the microemulsion in a dispersed liquid droplet form.

According to the present invention, in order to increase the oil amount carried in the microemulsion, the oil phase must contain at least one oil/fat component containing polyoxypropylene chains. Said oil/fat component containing polyoxypropylene chains can be for example polyoxypropylene fatty alcohol ethers, polyoxypropylene fatty acid esters and polyoxypropylene-co-polydimethylsiloxane, etc.

According to the present invention, as the oil/fat of polyoxypropylene fatty alcohol ethers, preference is given to polyoxypropylene fatty alcohol ethers, in which the fatty alcohol forming the polyoxypropylene fatty alcohol ether is saturated or unsaturated, and has 4-22, and preferably 8-18 carbon atoms, and the polyoxypropylene structure moiety comprises in average 2-60, and preferably 2-18 propylene oxide repeating units. Examples are polyoxypropylene-3 myristyl ether (TEGOSOFT® APM), polyoxypropylene-11 stearyl ether (TEGOSOFT® APS), polyoxypropylene-11 isostearyl ether, polyoxypropylene-14 butyl ether (TEGOSOFT® PVE), polyoxypropylene-15 stearyl ether (TEGOSOFT® E), polyoxypropylene-15 isostearyl ether, polyoxypropylene-7 lauryl ether, polyoxypropylene-10 oleyl ether, etc. Particularly, preference is given to polyoxypropylene fatty alcohol ethers, in which the fatty alcohol forming the polyoxypropylene fatty alcohol ether is saturated or unsaturated, and has 8-18 carbon atoms, and the polyoxypropylene structure moiety comprises in average 8-16 propylene oxide repeating units, and especially the polyoxypropylene fatty alcohol ethers, in which the fatty alcohol forming the polyoxypropylene fatty alcohol ether is saturated or unsaturated, and has 12-18 carbon atoms, and the polyoxypropylene structure moiety comprises in average 10-16 propylene oxide repeating units, such as polyoxypropylene-11 stearyl ether (TEGOSOFT® APS) and/or polyoxypropylene-15 stearyl ether (TEGOSOFT® E).

According to the present invention, as the oil/fat of polyoxypropylene fatty acid esters, preference is given to polyoxypropylene fatty acid esters, in which the fatty acid forming the polyoxypropylene fatty acid ester is saturated or unsaturated, and has 8-22, and preferably 8-18 carbon atoms, and the polyoxypropylene structure moiety comprises in average 2-60, and preferably 6-26 propylene oxide repeating units. Examples are polyoxypropylene-15 stearate, polyoxypropylene-15 isostearate, polyoxypropylene-26 oleate (supplied by BASF), polyoxypropylene-9 laurate (supplied by A&E Connock), polyoxypropylene-6 ricinoleate, etc. Particularly, preference is given to polyoxypropylene fatty acid esters, in which the fatty acid forming the polyoxypropylene fatty acid ester is saturated or unsaturated, and has 8-18 carbon atoms, and the polyoxypropylene structure moiety comprises in average 8-16 propylene oxide repeating units, and especially the polyoxypropylene fatty acid esters, in which the fatty acid forming the polyoxypropylene fatty acid ester is saturated or unsaturated, and has 12-16 carbon atoms, and the polyoxypropylene structure moiety comprises in average 8-12 propylene oxide repeating units, such as polyoxypropylene-9 laurate (supplied by A&E Connock).

According to the present invention, as the oils of polyoxypropylene-co-polydimethylsiloxanes, preference is give to polyoxypropylene-co-polydimethylsiloxane, in which the polyoxypropylene structure moiety comprises in average 2-60, and preferably 2-30 propylene oxide repeating units. Examples are polyoxypropylene-2-co-polydimethylsiloxane (PPG-2 Dimethicone, supplied by Dow-Corning), polyoxypropylene-12-co-polydimethylsiloxane (PPG-12 Dimethicone, supplied by GE), polyoxypropylene-27-co-polydimethylsiloxane (supplied by Shin Etsu), etc. Particularly, preference is give to polyoxypropylene-co-polydimethylsiloxanei, in which the polyoxypropylene structure moiety comprises in average 2-6 propylene oxide repeating units, such as polyoxypropylene-2-co-polydimethylsiloxane (supplied by Dow-Corning).

In the microemulsion of the present invention, as said oil/fat component containing polyoxypropylene chains, use can be made of polyoxypropylene fatty alcohol ethers, or polyoxypropylene fatty acid esters, or polyoxypropylene-co-polydimethylsiloxane; in addition, use can also be made of any combination of two or more selected from a polyoxypropylene fatty alcohol ether, a polyoxypropylene fatty acid ester, and polyoxypropylene-co-polydimethylsiloxane; and for example, use can be made of a combination of polyoxypropylene-2-co-polydimethylsiloxane (supplied by Dow-Corning) with polyoxypropylene-11 stearyl ether (TEGOSOFT® APS).

In addition to the oil/fat containing polyoxypropylene chains, ordinary hydrophobic components, or oil phase components, or all of the oil components which are allowed to be used in the cosmetics can also be used in the present invention. These oil phase components can be at least one oil/fat selected form vegetable oil, mineral oil, silicon oil and synthesized oil that are traditionally used, and can also be various waxes that are traditionally used.

The oil/fat such as silicon oil suitable for the present invention is, for example, polydimethylsiloxane and cyclomethylsiloxane, and also aryl-, alkyl- or alkoxy-substituted polymethylsiloxanes and cyclomethylsiloxanes.

The oil/fat suitable for the present invention also comprises mono- or diesters of linear and/or branched mono- and/or dicarboxylic acids having 2 to 44 carbon atoms with saturated or unsaturated, linear and/or branched alcohols having 1 to 22 carbon atoms. Likewise, as the oil/fat used in the present invention, use can also be made of the esters of bifunctional aliphatic alcohols having 2-36 carbon atoms with monofunctional aliphatic carboxylic acids having 1-22 carbon atoms.

As the oil/fat used in the present invention, use can particularly also be made of esters of fatty acids having 12-22 carbon atoms, such as methyl esters and isopropyl esters, such as methyl laurate, methyl stearate, methyl oleate, methyl erucate, isopropyl palmitate, isopropyl myristate, isopropyl stearate and/or isopropyl oleate.

In addition, as the oil/fat used in the present invention, particularly preference is also given to n-butyl stearate, n-hexyl laurate, n-decyl oleate, isooctyl stearate, isononyl palmitate, isononyl isononanoate, 2-ethylhexyl palmitate, 2-ethylhexyl laurate, 2-hexyl-decyl stearate, 2-octyldodecyl palmitate, oleyl oleate, oleyl erucate and/or erucyl oleate.

As the oil/fat used in the present invention, dicarboxylic acid esters are also especially suitable, e.g., di(n-butyl) adipate, di(n-butyl) sebacate, di(2-ethylhexyl) adipate, di(2-hexyldecyl) succinate and/or diiso-tridecyl azelate. As the oil/fat used in the present invention, diol esters are also especially suitable, e.g., ethylene glycol dioleate, ethylene glycol diisotri-decanoate, propylene glycol di(2-ethylhexanoate), butanediol diisostearate and/or neopentyl glycol dicaprylate.

As the oil/fat used in the present invention, use can also be made of carboxylic acid diesters, such as di(2-ethylhexyl) carbonate.

It is also suitable to use relatively long-chain (C₁₀ to C₃₀) triglycerides, i.e. triple esters of glycerol with three acid molecules, at least one of which is a relatively long-chain acid molecule. Mention can be made here, by way of example, of fatty acid triglycerides, which comprise synthetic triglycerides of caprylic/capric acid mixtures, triglycerides of industrial oleic acids, triglycerides of isostearic acids and triglycerides of palmitic/oleic acid mixtures. Additionally use can also be made of linear or branched fatty alcohols, such as oleyl alcohol or octyldodecanol, and also fatty alcohol ethers, such as dioctyl ether, etc.

As the oil/fat used in the present invention, it is also suitable to use, for example, natural vegetable oils, e.g. olive oil, sunflower oil, soybean oil, peanut oil, rapeseed oil, almond oil, palm oil and/or jojoba oil, and also the liquid portion of coconut oil or palm kernel oil, and also the liquid portions of animal oils, such as sperm oil, neatsfoot oil or beef tallow.

As the oil/fat of the present invention, use can further be made of hydrocarbon oil/fat, particularly liquid paraffin and isoparaffin. Examples of hydrocarbon oil/fat which can be used are paraffin oil, white mineral oil, isohexadecane, polydecene, petroleum jelly, light liquid paraffin and/or squalane. Furthermore, esters of arylcarboxylic acids are also suitable, such as, esters of benzoic acid, e.g. benzoic acid esters formed by saturated or unsaturated and linear or branched alcohols having 1 to 22 carbon atoms with benzoic acid, such as isostearyl benzoate and 2-octyldodecyl benzoate, preferably C12-15-alkyl benzoate.

In a preferred embodiment of the present invention, in addition to the oil/fat containing polyoxypropylene chains, preferably the oil phase also comprises one or more of oil/fat components selected from the following group of: cetyl polydimethylsiloxane (ABIL^{®} WAX9801), 2-ethylhexyl palmitate (TEGOSOFT^{®} OP), di(2-ethylhexyl) carbonate (TEGOSOFT^{®} DEC), cyclomethylsiloxane, polydimethylsiloxane (ABIL^{®} 350), white mineral oil and cyclomethylsiloxane/dimethylsilanol (ABIL^{®} OSW5).

In the microemulsion of the present invention, the amount of the oil phase used as the component B is usually 0.1-20 wt%, preferably 0.5-10 wt%, and more preferably 1-8 wt%, based on the total weight of the microemulsion.

In the oil phase of the microemulsion in the present invention, the content of the oil/fat component containing polyoxylpropylene chains as a necessary component accounts for 20-100 wt% of the total amount of the oil phase, and preferably accounts for 50-100 wt% of the total weight of the oil phase.

### Component C

In the microemulsion of the present invention, a surfactant or an emulsifier is also included, which is an oil-in-water nonionic emulsifier; preferably it is one or more hydrophilic nonionic surfactants selected from the following group of hydrophilic nonionic surfactants with polyoxyethylene chains as hydrophilic groups, polyglycerol fatty acid esters, polyglycerol fatty alcohol ethers, sucrose fatty acid esters, and hydrocarbyl polyglycosides.

In a preferred embodiment of the present invention, the oil-in-water nonionic emulsifier as component C is a two-component composite emulsifier composed of an emulsifier X and an emulsifier Y, in which the emulsifier X is a hydrophilic nonionic surfactant with the polyoxyethylene chains as the hydrophilic groups, while component Y is a hydrophilic nonionic surfactant selected from a polyglycerol fatty acid ester, a polyglycerol fatty alcohol ether, a sucrose fatty acid ester or a hydrocarboxyl polyglycoside.

According to the present invention, under the condition of a composite emulsifier being used, based on the total weight of the emulsifier, the amount of the emulsifier X used is 50-90 wt%, and the amount of the emulsifier Y used is 10-50 wt%; and preferably, the amount of the emulsifier X used is 65-90 wt%, and the amount of the emulsifier Y used is 10-35 wt%.

In a preferred embodiment of the present invention, said hydrophilic nonionic surfactant with polyoxyethylene chains as hydrophilic groups is selected from the following group of polyoxyethylene fatty acid esters, polyoxyethylene fatty alcohol ethers, polyoxyethylene sorbitol anhydride fatty acid esters, polyoxyethylene glycerol mono fatty acid esters, polyoxyethylene hydrogenated castor oil and polyoxyethylene hydrogenated castor oil mono fatty acid esters.

More particularly, as the above polyoxyethylene fatty acid esters, preference is give to the polyoxyethylene fatty acid esters, in which the fatty acid forming the polyoxyethylene fatty acid ester is saturated or unsaturated, and has 8-22 carbon atoms, and the polyoxyethylene structure moiety comprises in average 4-20 ethylene oxide repeating units. Examples are polyoxyethylene caprylate, polyoxyethylene 2-ethyl hexanoate, polyoxyethylene caprate, polyoxyethylene laurate, polyoxyethylene myristate, polyoxyethylene palmitate, polyoxyethylene isostearate, polyoxyethylene stearate, polyoxyethylene oleate, polyoxyethylene behenate, etc. A polyoxyethylene fatty acid ester is particularly preferred, in which the fatty acid forming the polyoxyethylene fatty acid ester is saturated or unsaturated and has 8-18 carbon atoms and the polyoxyethylene structure moiety comprises in average 4-12 ethylene oxide repeating units, such as polyoxyethylene-6 caprylate/caprate (TEGOSOFT^{®} GMC 6), polyoxyethylene-6 laurate, polyoxyethylene-8 laurate and/or polyoxyethylene-12 palmitate. It is especially a polyoxyethylene fatty acid ester, in which the fatty acid forming the polyoxyethylene fatty acid ester is saturated or unsaturated and has 8-12 carbon atoms and the polyoxyethylene structure moiety comprises in average 4-8 ethylene oxide repeating units, such as polyoxyethylene-6 caprylate/caprate (TEGOSOFT^{®} GMC 6).

More particularly, as the above polyoxyethylene fatty alcohol ethers, preference is given to the polyoxyethylene fatty alcohol ether, in which the fatty alcohol forming the polyoxyethylene fatty alcohol ether is saturated or unsaturated, and has 8-22 carbon atoms, and the polyoxyethylene structure moiety comprises in average 4-60 ethylene oxide repeating units. As examples thereof, reference can be made of polyoxyethylene octyl ethers, polyoxyethylene decyl ethers, polyoxyethylene lauryl ethers, polyoxyethylene myristyl ethers, polyoxyethylene palmityl ethers, polyoxyethylene isostearyl ethers, polyoxyethylene stearyl ethers, polyoxyethylene oleyl ethers, polyoxyethylene behenyl ethers, etc. Particularly preference is given to the polyoxyethylene fatty alcohol ethers, in which the fatty alcohol forming the polyoxyethylene fatty alcohol ether is saturated or unsaturated and has 8-18 carbon atoms, and the polyoxyethylene structure moiety comprises in average 4-12 ethylene oxide repeating units, such as polyoxyethylene-8 lauryl ether and/or polyoxyethylene-5 octyl ether. Especially, preference is given to the polyoxyethylene fatty alcohol ethers, in which the fatty alcohol forming the polyoxyethylene fatty alcohol ether is saturated or unsaturated and has 10-14 carbon atoms, and the polyoxyethylene structure moiety comprises in average 6-10 ethylene oxide repeating units, such as polyoxyethylene 8-lauryl ether.

More particularly, as the above polyoxyethylene sorbitol anhydride fatty acid ester, preference is given to the polyoxyethylene sorbitol anhydride fatty acid esters, in which the fatty acid forming the polyoxyethylene sorbitol anhydride fatty acid ester is saturated or unsaturated and has 8-22 carbon atoms, and the polyoxyethylene structure moiety comprises in average 4-60 ethylene oxide repeating units, such as polyoxyethylene sorbitol anhydride mono-, di-, and tri-fatty acid ester. Examples are polyoxyethylene sorbitol anhydride mono-, di-, and tri-caprylate, a polyoxyethylene sorbitol anhydride mono-, di-, and tri-caprate, a polyoxyethylene sorbitol anhydride mono-, di-, and tri-laurate, a polyoxyethylene sorbitol anhydride mono-, di-, and tri-myristate, a polyoxyethylene sorbitol anhydride mono-, di-, and tri-palmitate, a polyoxyethylene sorbitol anhydride mono-, di-, and tri-isostearate, a polyoxyethylene sorbitol anhydride mono-, di-, and tri-stearate, a polyoxyethylene sorbitol anhydride mono-, di-, and tri-oleate, a polyoxyethylene sorbitol anhydride mono-, di-, and tri-behenate, etc. Particularly, preference is given to the polyoxyethylene sorbitol anhydride fatty acid esters, in which the fatty acid forming the polyoxyethylene sorbitol anhydride fatty acid ester is saturated or unsaturated and has 8-18 carbon atoms, and the polyoxyethylene structure moiety comprises in average 4-12 ethylene oxide repeating units, such as polyoxyethylene sorbitol anhydride mono-, di-, and tri-fatty acid esters.

More particularly, as the above polyoxyethylene glycol mono fatty acid esters, preference is given to the polyoxyethylene glycol mono fatty acid esters, in which the fatty acid forming the polyoxyethylene glycol mono fatty acid ester is saturated or unsaturated, and has 8-22 carbon atoms, and the polyoxyethylene structure moiety comprises in average 4-60 ethylene oxide repeating units. Examples are polyoxyethylene glycerol mono-caprylate, polyoxyethylene glycerol mono-caprate, polyoxyethylene glycerol mono-laurate, polyoxyethylene glycerol mono-myristate, polyoxyethylene glycerol mono-palmitate, polyoxyethylene glycerol mono-isostearate, polyoxyethylene glycerol mono-stearate, polyoxyethylene glycerol mono-oleate, polyoxyethylene glycerol mono-behenate, etc. Particularly, preference is given to the polyoxyethylene glycerol mono fatty acid esters, in which the fatty acid forming the polyoxyethylene glycerol mono fatty acid ester is saturated or unsaturated and has 8-18 carbon atoms and the polyoxyethylene structure moiety comprises in average 4-12 ethylene oxide repeating units.

More particularly, as the above polyoxyethylene hydrogenated castor oil, preference is given to the polyoxyethylene hydrogenated castor oil, in which the polyoxyethylene structure moiety comprises in average 20-80, and preferably 30-60 ethylene oxide repeating units.

More particularly, as the above polyoxyethylene hydrogenated castor oil mono fatty acid esters, preference is given to the polyoxyethylene hydrogenated castor oil mono fatty acid esters, in which the fatty acid forming the polyoxyethylene hydrogenated castor oil mono fatty acid ester is saturated or unsaturated, and has 8-22 carbon atoms, and the polyoxyethylene structure moiety comprises in average 4-60 ethylene oxide repeating units. As examples thereof, reference can be made of polyoxyethylene hydrogenated castor oil mono-caprylate, polyoxyethylene hydrogenated castor oil mono-caprate, polyoxyethylene hydrogenated castor oil mono-laurate, polyoxyethylene hydrogenated castor oil mono-myristate, polyoxyethylene hydrogenated castor oil mono-palmitate, polyoxyethylene hydrogenated castor oil mono-isostearate, polyoxyethylene hydrogenated castor oil mono-stearate, polyoxyethylene hydrogenated castor oil mono-oleate, polyoxyethylene hydrogenated castor oil mono-behenate, etc. Particularly, preference is given to the polyoxyethylene hydrogenated castor oil mono fatty acid esters, in which the fatty acid forming the polyoxyethylene hydrogenated castor oil mono fatty acid ester is saturated or unsaturated and has 8-18 carbon atoms and the polyoxyethylene structure moiety comprises in average 4-12 ethylene oxide repeating units.

More particularly, as the above polyglycerol fatty acid ester, preference is given to the polyglycerol fatty acid esters, in which the fatty acid forming the polyglycerol fatty acid ester is saturated or unsaturated and has 8-22 carbon atoms, and the polyglycerol structure moiety comprises in average 2-60 glycerol repeating units; and the polyglycerol fatty acid esters can be a polyglycerol mono fatty acid ester or can be a polyglycerol poly fatty acid ester. Examples are polyglycerol mono- and polycaprylate, polyglycerol mono- and poly2-ethyl hexanoate, polyglycerol mono-and polycaprate, polyglycerol mono- and polylaurate (such as polyglycerol-10 pentalaurate), polyglycerol mono- and polymyristate, polyglycerol mono- and poly palmitate, polyglycerol mono- and polyisostearate, polyglycerol mono- and poly stearate (such as polyglycerol-3 distearate), polyglycerol mono- and polyoleate (such as polyglycerol-2 dioleate), polyglycerol mono- and polybehenate, etc. Particularly, preference is given to the polyglycerol mono and/or poly fatty acid esters, in which the fatty acid forming the polyglycerol fatty acid ester is saturated or unsaturated and has 8-18 carbon atoms, and the polyglycerol structure moiety comprises in average 3-15 glycerol repeating units, such as polyglycerol-3 mono caprate (TEGOSOFT^{®} PC31), polyglycerol-4 mono caprate (TEGOSOFT^{®} PC41), polyglycerol-3 mono caprylate (TEGO^{®} COSMO P813) and/or polyglycerol-4 mono laurate (TEGO^{®} CARE PL 4). It is especially a polyglycerol mono and/or poly fatty acid ester, in which the fatty acid forming the polyglycerol fatty acid ester is saturated or unsaturated and has 8-12 carbon atoms, and the polyglycerol structure moiety comprises in average 3-6 glycerol repeating units; such as polyglycerol-3 mono caprate (TEGOSOFT^{®} PC31), and/or polyglycerol-4 mono caprate (TEGOSOFT^{®} PC41).

More particularly, as the above polyglycerol fatty alcohol ethers, preference is given to the polyglycerol fatty alcohol ethers, in which the fatty alcohol forming the polyglycerol fatty alcohol ether is saturated or unsaturated, and has 8-22 carbon atoms, and the polyglycerol structure moiety comprises in average 2-60 glycerol repeating units. As examples thereof, reference can be made of: polyglycerol octyl ether, polyglycerol decyl ether, polyglycerol lauryl ether, polyglycerol myristyl ether, polyglycerol palmityl ether, polyglycerol isostearyl ether, polyglycerol stearyl ether, polyglycerol oleyl ether, polyglycerol behenyl ether, etc. Particularly, preference is given to the polyglycerol fatty alcohol ethers, in which the fatty alcohol forming the polyglycerol fatty alcohol ether is saturated or unsaturated, and has 8-18 carbon atoms, and the polyglycerol structure moiety comprises in average 3-15 glycerol repeating units, especially the polyglycerol fatty alcohol ethers, in which the fatty alcohol forming the polyglycerol fatty alcohol ether is saturated or unsaturated, and has 10-14 carbon atoms, and the polyglycerol structure moiety comprises in average 3-5 glycerol repeating units, such as polyglycerol-4 lauryl ether.

More particularly, as the above sucrose fatty acid esters, preference is given to the sucrose fatty acid esters, in which the fatty acid forming the sucrose fatty acid ester is saturated or unsaturated, and has 8-22 carbon atoms, the sucrose fatty acid ester can be either a sucrose mono fatty acid ester, or a sucrose poly fatty acid ester, such as sucrose di-, tri-, tetra-, penta-, and hexa-fatty acid ester. As examples thereof, reference can be made of: sucrose mono- and polycaprylate, sucrose mono- and poly 2-ethyl hexanoate, sucrose mono- and polycaprate, sucrose mono- and polylaurate (such as sucrose dilaurate), sucrose mono- and polymyristate, sucrose mono- and polypalmitate (such as sucrose hexapalmitate), sucrose mono- and polyisostearate, sucrose mono- and polystearate, sucrose mono- and polyoleate, sucrose mono- and polybehenate, etc. Particularly, preference is given to the sucrose mono- and/or poly fatty acid esters, in which the fatty acid forming the sucrose fatty acid ester is saturated or unsaturated, and has 8-18 carbon atoms, especially a sucrose mono-and/or poly fatty acid ester, in which the fatty acid forming the sucrose fatty acid ester is saturated or unsaturated, and has 12-18 carbon atoms, such as sucrose mono-and/or dilaurate and/or sucrose mono- and/or distearate.

More particularly, as the above hydrocarbyl polyglycosides, preference is given to the hydrocarbyl polyglucosides, in which the hydrocarbyl is saturated or unsaturated, linear or branched and has 8-22 carbon atoms, and the average degree of condensation of the glycoside unit is 1-7, and more preferably the hydrocarbyl polyglucosides, in which the hydrocarbyl is saturated or unsaturated, linear or branched and has 8-22 carbon atoms, and the average degree of condensation of the glycoside unit being 1-7, and the glycoside being glucoside. Examples are octyl polyglycoside (glucoside), 2-ethylhexyl polyglycoside (glucoside), decyl polyglycoside (glucoside), lauryl polyglycoside (glucoside), myristyl polyglycoside (glucoside), palmityl polyglycoside (glucoside), isostearyl polyglycoside (glucoside), stearyl polyglycoside (glucoside), oleyl polyglycoside (glucoside), behenyl polyglycoside (glucoside), etc. Particularly, preference is given to the hydrocarbyl polyglucosides, in which the hydrocarbyl is saturated or unsaturated, linear or branched and has 8-11 carbon atoms, the average degree of condensation of the glycoside unit being 1-1.4, and the glycoside being glucoside, and/or a hydrocarbyl polyglucoside in which the hydrocarbyl is saturated or unsaturated, linear or branched and has 12-14 carbon atoms, and the average degree of condensation of the glycoside unit being 1.5-4.0, and the glycoside being glucoside.

The two component composite emulsifier suitable for the microemulsion of the present invention is preferably a composite emulsifier composed of a polyoxyethylene fatty acid ester and a polyglycerol fatty acid ester, such as a composite emulsifier composed of polyoxyethylene-6 caprylate/caprate (TEGOSOFT^{®} GMC 6), with polyglycerol-3 mono caprate (TEGOSOFT^{®} PC31) or with polyglycerol-4 mono caprate (TEGOSOFT^{®} PC41). In addition, the two component composite emulsifier suitable for the microemulsion of the present invention is also preferably a composite emulsifier composed of a polyoxyethylene fatty alcohol ether and a polyglycerol fatty alcohol ether, such as a composite emulsifier composed of polyoxyethylene-8 lauryl ether and polyglycerol-4 lauryl ether.

In the microemulsion of the present invention, the amount of the emulsifier used as the component C is usually 1-25 wt%, preferably 1-15 wt%, and more preferably 2-10 wt%, based on the total weight of the microemulsion.

In the oil-in-water microemulsion of the present invention, it can also comprise a component commonly used in the cosmetics, such as, in addition to the emulsifier or surfactant described above, a tackifier, a sterilizing agent, a humectant, a wetting agent, a colourant, a preservative, a feel improving agent, a fragrance, an antiinflammatory, a whitening agent, an antiperspirant, an ultra-violet absorbent, etc.

As mentioned above, by including the oil/fat containing polyoxypropylene chains in the oil-in-water microemulsion, not only the oil amount carried in the microemulsion is increased, but also the amount of the emulsifier used is reduced. Therefore, in a preferred embodiment of the present invention, the weight ratio of the oil phase to the oil-in-water phase nonionic emulsifier is above 0.25, and more preferably 0.5-1.

Therefore, the present invention also relates to the use of oil/fat containing polyoxypropylene chains as described above in increasing the amount of oil carried in an oil-in-water microemulsion.

Unless otherwise specified, the percentage contents mentioned in the present invention refer to the weight percentage contents.

The oil-in-water microemulsion of the present invention can be prepared by conventional processes. Generally speaking, each of the components in the microemulsion formulation of the present invention is added into a container, and mixed under stirring, if necessary by heating, and the oil-in-water microemulsion of the present invention can be obtained.

The oil-in-water microemulsion of the present invention has a transparent or translucent appearance, and has an average particle size of about 20-100 nm tested by a particle size distribution analyzer Model: DELSA NANO S from the BECKMAN COULTER company.

The present invention is explained hereinbelow by embodiments, but these embodiments are not to be interpreted as limiting the scope of the present invention.

### Embodiments

All components listed in the embodiments and the comparative example of the present invention are mixed under stirring; when the formulation contains a component in a solid or gel state at room temperature, it is necessary to heating the mixed system until each component is completely dissolved under stirring; then it is cooled; and the microemulsion of the present invention or the comparative mixture product is obtained. Each product obtained from the comparative example and the embodiments is tested by a particle size distribution analyzer Model: DELSA NANO S from the BECKMAN COULTER company.

### Comparative example 1

The formulation of comparative example 1 is shown in Table 1c.

**Table 1c**

| component | wt% |
|---|---|
| polyoxyethylene-6 caprylate/caprate (TEGOSOFT^{®} GMC 6) | 4 |
| polyglycerol-3 mono caprate (TEGOSOFT^{®} PC31) | 4 |
| 2-ethylhexyl palmitate (TEGOSOFT^{®} OP) | 2 |
| preservative | 0.1 |
| water | top up to 100 |

The product obtained from comparative example 1 (product 1c) had an average particle size of about 188 nm, a cloudy appearance, and did not form a microemulsion. For this mixed system, the weight ratio of the oil phase which was able to be emulsified to the emulsifier was lower than 0.25, and therefore the oil amount carried was lower than 2 wt%.

### Embodiment 1

The formulation of embodiment 1 is shown in Table 1.

**Table 1**

| component | wt% |
|---|---|
| polyoxyethylene-6 caprylate/caprate (TEGOSOFT^{®} GMC 6) | 4 |
| polyglycerol-3 mono caprate (TEGOSOFT^{®} PC31) | 2 |
| 2-ethylhexyl palmitate (TEGOSOFT^{®} OP) | 1.6 |
| polyoxypropylene-11 stearyl ether (TEGOSOFT^{®} APS) | 0.4 |
| preservative | 0.1 |
| water | top up to 100 |

20 wt% of 2-ethylhexyl palmitate used in comparative example 1 was replaced by polyoxypropylene-11 stearyl ether (TEGOSOFT^{®} APS). The emulsified product obtained from embodiment 1 (product 1) had an average particle size of about 84 nm, a transparent appearance, and formed a microemulsion. For this microemulsion, the weight ratio of the oil phase to the emulsifier was 2/6 = 0.33, and the actual oil content was 2 wt%.

From the comparison between comparative example 1 and embodiment 1, when polyoxypropylene-11 stearyl ether was used as an oil/fat component in the oil phase, it was able to achieve a good emulsion effect even under the condition that the amount of the emulsion used was reduced. It can be seen here that for a conventional microemulsion formulation, when a small amount of oil phase component is replaced by oil/fat of a polyoxypropylene fatty alcohol ether type, the amount of the emulsifier used can be significantly reduced. Moreover, compared with the product of the comparative example 1, the product obtained from embodiment 1 not only had a transparent appearance, but also had relatively weak sticky feel in use, thus improving the skin feel significantly.

### Embodiment 2

The formulation of embodiment 2 is shown in Table 2.

**Table 2**

| component | wt% |
|---|---|
| polyoxyethylene-6 caprylate/caprate (TEGOSOFT^{®} GMC 6) | 4 |
| polyglycerol-3 mono caprate (TEGOSOFT^{®} PC31) | 4 |
| 2-ethylhexyl palmitate (TEGOSOFT^{®} OP) | 2 |
| polyoxypropylene-11 stearyl ether (TEGOSOFT^{®} APS) | 1 |
| preservative | 0.1 |
| water | top up to 100 |

The only difference of the formulation in this embodiment and that of the comparative 1 was in that 1 wt% of water used in comparative example 1 was replaced by 1 wt% polyoxypropylene-11 stearyl ether (TEGOSOFT^{®} APS). The emulsified product obtained from embodiment 2 (product 2) had an average particle size of about 47 nm, a transparent appearance, and formed a microemulsion. For this microemulsion, the weight ratio of the oil phase to the emulsifier was 3/8 = 0.375, and the actual oil content was 3 wt%.

It can be seen here that, for a conventional microemulsion formulation, when an oil/fat of a polyoxypropylene fatty alcohol ether is used in the oil phase, the amount of oil carried in this microemulsion can be increased significantly. Moreover, compared with the product of the comparative example 1, the product obtained from embodiment 2 not only had a transparent appearance, but also had relatively weak sticky feel in use, thus improving the skin feel significantly.

### Embodiment 3

The formulation of embodiment 3 is shown in Table 3.

**Table 3**

| component | wt% |
|---|---|
| polyoxyethylene-6 caprylate/caprate (TEGOSOFT^{®} GMC 6) | 5.8 |
| polyglycerol-3 mono caprate (TEGOSOFT^{®} PC31) | 2.2 |
| polyoxypropylene-11 stearyl ether (TEGOSOFT^{®} APS) | 6 |
| preservative | 0.1 |
| water | top up to 100 |

In this embodiment, the whole oil phase component was polyoxypropylene-11 stearyl ether. The emulsified product obtained from embodiment 3 (product 3) had an average particle size of about 56 nm, a transparent appearance, and formed a microemulsion. For this microemulsion, the weight ratio of the oil phase to the emulsifier was 6/8 = 0.75, and the actual oil content was 6 wt%.

It can be seen here that when polyoxypropylene-11 stearyl ether is used as the oil/fat component in the oil phase, the oil amount carried can be significantly increased under the condition that the amount of the emulsifier used is not changed. Moreover, compared with the product of the comparative example 1, the product obtained from embodiment 3 not only had a transparent appearance, but also had relatively weak sticky feel in use, thus improving the skin feel significantly.

### Embodiment 4

The formulation of embodiment 4 is shown in Table 4.

**Table 4**

| component | wt% |
|---|---|
| polyoxyethylene-6 caprylate/caprate (TEGOSOFT^{®} GMC 6) | 6.8 |
| polyglycerol-4 mono caprate (TEGOSOFT^{®} PC41) | 1.2 |
| polyoxypropylene-9 laurate (supplied by A&E Connock) | 2 |
| glycerol | 1 |
| preservative | 0.1 |
| water | top up to 100 |

The emulsified product obtained from embodiment 4 (product 4) had an average particle size of about 76 nm, a transparent appearance, and formed a microemulsion. For this microemulsion, the weight ratio of the oil phase to the emulsifier was 2/8 = 0.25, and the actual oil content was 2 wt%.

This embodiment also shows that the use of polyoxypropylene-9 laurate as the oil/fat component in the oil phase can increase the oil amount carried in the microemulsion. Moreover, compared with the product of the comparative example 1, the product obtained from embodiment 4 not only had a transparent appearance, but also had relatively weak sticky feel in use, thus improving the skin feel significantly.

### Embodiment 5

The formulation of embodiment 5 is shown in Table 5.

**Table 5**

| component | wt% |
|---|---|
| polyoxyethylene-8 lauryl ether | 5.8 |
| polyglycerol-4 lauryl ether | 2.2 |
| polyoxypropylene-2-co-polydimethylsiloxane (supplied by Dow-Corning) | 1.5 |
| polydimethylsiloxane (ABIL^{®} 350) | 0.5 |
| hyaluronic acid | 0.5 |
| preservative | 0.1 |
| water | top up to 100 |

The emulsified product obtained from embodiment 5 (product 5) had an average particle size of about 65 nm, a transparent appearance, and formed a microemulsion. For this microemulsion, the weight ratio of the oil phase to the emulsifier was 2/8 = 0.25, and the actual oil content was 2 wt%.

This embodiment also shows that using polyoxypropylene-2-co-polydimethylsiloxane as the oil/fat component in the oil phase can increase the oil amount carried in the microemulsion. Moreover, compared with the product of the comparative example 1, the product obtained from embodiment 5 not only had a transparent appearance, but also had relatively weak sticky feel in use, thus improving the skin feel significantly.

### Embodiment 6

The formulation of embodiment 6 is shown in Table 6.

**Table 6**

| component | wt% |
|---|---|
| polyoxyethylene-6 caprylate/caprate (TEGOSOFT^{®} GMC 6) | 17 |
| polyglycerol-3 mono caprate (TEGOSOFT^{®} PC31) | 8 |
| polyoxypropylene-15 stearyl ether (TEGOSOFT^{®} E) | 20 |
| water | top up to 100 |

The emulsified product obtained from the embodiment (product 6) had an average particle size of about 50 nm, a transparent appearance, and formed a microemulsion. For this microemulsion, the weight ratio of the oil phase to the emulsifier was 20/25 = 0.8, and the actual oil content was 20 wt%.

This embodiment also shows that, when polyoxypropylene-15 stearyl ether is used as the oil/fat component in the oil phase, the oil amount carried can be increased, but such high oil amount carried is not achievable by the conventional microemulsions.

### Thermal stability test

Products 1-6 obtained from the above embodiments 1-6 were each subjected to a thermal stability test. The stability test at room temperature refers to storing the samples at 20-25°C for three months. As a result, after having been subjected to the above tests, the appearances of the products 1-6 without one exception, remained to be in the same transparent state as at the beginning of the test, and none of the phenomena of discoloration, cloudiness, etc. occurred. It was shown thereby that products 1-6 were stable.

## Claims

1. An oil-in-water microemulsion comprising, on the bases of its total weight, the following components of:
A) 55-95 wt% of a water phase;
B) 0.1-20 wt% of an oil phase, wherein the oil phase comprises at least one oil/fat component containing polyoxypropylene chains; and
C) 1-25 wt% of an oil-in-water nonionic emulsifier, wherein the emulsifier is one or more hydrophilic nonionic surfactants selected from the following group of hydrophilic nonionic surfactants with polyoxyethylene chains as hydrophilic groups, polyglycerol fatty acid esters, polyglycerol fatty alcohol ethers, sucrose fatty acid esters, and hydrocarbyl polyglycosides.

2. The oil-in-water microemulsion as claimed in claim 1, wherein, the oil/fat comprising polyoxypropylene chains is a polyoxypropylene fatty alcohol ether, a polyoxypropylene fatty acid ester, polyoxypropylene-co-polydimethylsiloxane or any mixture thereof.

3. The oil-in-water microemulsion as claimed in claim 1 or 2, wherein
as the oil/fat containing polyoxypropylene chains, the polyoxypropylene fatty alcohol ether is a polyoxypropylene fatty alcohol ether in which the fatty alcohol forming the polyoxypropylene fatty alcohol ether is saturated or unsaturated, and has 8-18 carbon atoms, and the polyoxypropylene structure moiety comprises in average 2-18 propylene oxide repeating units; preferably polyoxypropylene-3 myristyl ether, polyoxypropylene-11 stearyl ether, polyoxypropylene-11 isostearyl ether, polyoxypropylene-14 butyl ether, polyoxypropylene-15 stearyl ether, polyoxypropylene-15 isostearyl ether, polyoxypropylene-7 lauryl ether and/or polyoxypropylene-10 oleyl ether;
as the oil/fat containing polyoxypropylene chains, the polyoxypropylene fatty acid ester is a polyoxypropylene fatty acid ester in which the fatty acid forming the polyoxypropylene fatty acid ester is saturated or unsaturated, and has 8-18 carbon atoms, and the polyoxypropylene structure moiety comprises in average 6-26 propylene oxide repeating units; preferably polyoxypropylene-15 stearate, polyoxypropylene-15 isostearate, polyoxypropylene-26 oleate, polyoxypropylene-9 laurate and/or polyoxypropylene-6 ricinoleate; and
as the oil/fat containing polyoxypropylene chains, the polyoxypropylene-co-polydimethylsiloxane is polyoxypropylene-co-polydimethylsiloxane in which the polyoxypropylene structure moiety comprises in average 2-30 propylene oxide repeating units; preferably polyoxypropylene-2-co-polydimethylsiloxane, polyoxypropylene-12-co-polydimethylsiloxane and/or polyoxypropylene-27-co-polydimethylsiloxane.

4. The oil-in-water microemulsion as claimed in any of claims 1-3, wherein the oil/fat comprising polyoxypropylene chains accounts for 20-100 wt% of the total amount of the oil phase; and preferably accounts for 50-100 wt% of the total amount of the oil phase.

5. The oil-in-water microemulsion as claimed in any of claims 1-4, wherein the weight ratio of the oil phase to the oil-in-water nonionic emulsifier is above 0.25, and more preferably 0.5-1.

6. The oil-in-water microemulsion as claimed in any of claims 1-5, wherein based on the total weight of the microemulsion, the content of the oil phase is 0.5-10 wt%, and preferably 1-8 wt%; and the content of the oil-in-water nonionic emulsifier is 1-15 wt%, and preferably 2-10 wt%.

7. The oil-in-water microemulsion as claimed in any of claims 1-6, wherein the oil-in-water nonionic emulsifier as component C is a two-component composite emulsifier composed of an emulsifier X and an emulsifier Y, in which the component X is a hydrophilic nonionic surfactant with the polyoxyethylene chains as the hydrophilic groups, while the component Y is a hydrophilic nonionic surfactant selected from polyglycerol fatty acid esters, polyglycerol fatty alcohol ethers, sucrose fatty acid esters or hydrocarbyl polyglycosides.

8. The oil-in-water microemulsion as claimed in claim 7, wherein said two component composite emulsifier is a composite emulsifier composed of a polyoxyethylene fatty acid ester and a polyglycerol fatty acid ester, or a composite emulsifier composed of a polyoxyethylene fatty alcohol ether and a polyglycerol fatty alcohol ether, especially a composite emulsifier composed of polyoxyethylene-6 caprylate/caprate with polyglycerol-3 mono caprate or with polyglycerol-4 mono caprate, or a composite emulsifier composed of polyoxyethylene-8 lauryl ether and polyglycerol-4 lauryl ether.

9. The oil-in-water microemulsion as claimed in claim 7 or 8, wherein on the basis of the total amount of said composite emulsifier, the amount of the emulsifier X used is 50-90 wt%, and the amount of the emulsifier Y used is 10-50 wt%; and preferably the amount of the emulsifier X used is 65-90 wt%, and the amount of the emulsifier Y used is 10-35 wt%.

10. The oil-in-water microemulsion as claimed in any of claims 1-9, wherein said hydrophilic nonionic surfactant with polyoxyethylene chains as hydrophilic groups is selected from the following group of polyoxyethylene fatty acid esters, polyoxyethylene fatty alcohol ethers, polyoxyethylene sorbitol anhydride fatty acid esters, polyoxyethylene glycerol mono fatty acid esters, polyoxyethylene hydrogenated castor oil and polyoxyethylene hydrogenated castor oil mono fatty acid esters.

11. The oil-in-water microemulsion as claimed in any of claims 1-10, wherein
said polyoxyethylene fatty acid esters are polyoxyethylene fatty acid esters, in which the fatty acid forming the polyoxyethylene fatty acid esters is saturated or unsaturated and has 8-18 carbon atoms and the polyoxyethylene structure moiety comprises in average 4-12 ethylene oxide repeating units, preferably polyoxyethylene-6 caprylate/caprate, polyoxyethylene-6 laurate, polyoxyethylene-8 laurate and/or polyoxyethylene-12 palmitate;
said polyoxyethylene fatty alcohol ethers are polyoxyethylene fatty alcohol ethers, in which the fatty alcohol forming the polyoxyethylene fatty alcohol ethers is saturated or unsaturated and has 8-18 carbon atoms, and the polyoxyethylene structure moiety comprises in average 4-12 ethylene oxide repeating units, preferably polyoxyethylene-8 lauryl ether and/or polyoxyethylene-5 octyl ether;
said polyoxyethylene sorbitol anhydride fatty acid esters are polyoxyethylene sorbitol anhydride fatty acid esters, in which the fatty acid forming the polyoxyethylene sorbitol anhydride fatty acid ester is saturated or unsaturated and has 8-18 carbon atoms and the polyoxyethylene structure moiety comprises in average 4-12 ethylene oxide repeating units;
said polyoxyethylene glycerol mono fatty acid esters are polyoxyethylene glycerol mono fatty acid esters, in which the fatty acid forming the polyoxyethylene glycerol mono fatty acid ester is saturated or unsaturated and has 8-18 carbon atoms and the polyoxyethylene structure moiety comprises in average 4-12 ethylene oxide repeating units;
said polyoxyethylene hydrogenated castor oil is a polyoxyethylene hydrogenated castor oil, in which the polyoxyethylene structure moiety comprises in average 30-60 ethylene oxide repeating units;
said polyoxyethylene hydrogenated castor oil mono fatty acid esters are polyoxyethylene hydrogenated castor oil mono fatty acid esters, in which the fatty acid forming the polyoxyethylene hydrogenated castor oil mono fatty acid ester is saturated or unsaturated and has 8-18 carbon atoms, and the polyoxyethylene structure moiety comprises in average 4-12 ethylene oxide repeating units;
said polyglycerol fatty acid esters are polyglycerol mono and/or poly fatty acid esters, in which the fatty acid forming the polyglycerol fatty acid esters is saturated or unsaturated and has 8-18 carbon atoms, and the polyglycerol structure moiety comprises in average 3-15 glycerol repeating units; preferably polyglycerol-3 mono caprate, polyglycerol-4 mono caprate, polyglycerol-3 mono caprylate and/or polyglycerol-4 mono laurate;
said polyglycerol fatty alcohol ethers are polyglycerol fatty alcohol ethers, in which the fatty alcohol forming the polyglycerol fatty alcohol ethers is saturated or unsaturated, and has 8-18 carbon atoms, and the polyglycerol structure moiety comprises in average 3-15 glycerol repeating units, preferably polyglycerol-4 lauryl ether;
said sucrose fatty acid esters are sucrose mono- and/or poly fatty acid esters, in which the fatty acid forming the sucrose fatty acid esters is saturated or unsaturated, and has 8-18 carbon atoms; and
said hydrocarbyl polyglycosides are hydrocarbyl polyglucosides in which the hydrocarbyl is saturated or unsaturated, linear or branched and has 8-11 carbon atoms, the average degree of condensation of the glycoside units is 1-1.4, and the glycoside is glucoside, and/or are hydrocarbyl polyglucosides in which the hydrocarbyl is saturated or unsaturated, linear or branched and has 12-14 carbon atoms, the average degree of condensation of the glycoside units is 1.5-4.0, and the glycoside is glucoside.

12. Use of oil/fat containing polyoxypropylene chains as defined in any of claims 1-3 for increasing the amount of oil carried in an oil-in-water microemulsion.
